# EUROPEAN PATENT APPLICATION

(11) **EP 4 513 192 A1**
(43) Date of publication of application: **26.02.2025**
(21) Application number: 23791662.2
(22) Date of filing: 04.04.2023
(51) Int. Cl.: G01N 35/00

(54) **AUTOMATIC ANALYSIS SYSTEM AND MOBILE TERMINAL**

(30) Priority: 18.04.2022 JP 2022068135
(71) Applicant: HITACHI HIGH-TECH CORPORATION, Tokyo 105-6409 (JP)
(72) Inventor: KAWAKAMI Rimi, Tokyo 105-6409 (JP); TANOUE Hidetsugu, Tokyo 105-6409 (JP); SHIMODA Akihiro, Tokyo 105-6409 (JP); OBARI Kouichi, Tokyo 105-6409 (JP)
(74) Representative: Strehl Schübel-Hopf & Partner
(86) International application number: PCT/JP2023/013874
(87) International publication number: WO 2023/204011

(57) **Abstract**

The purpose of the present invention is to provide an automatic analysis system and a mobile terminal that make it possible to display information that a user wants to know through easier operation, without making any major changes to the hardware configuration of an analyzer. To this end, an automatic analysis system according to the present invention comprises: an automatic analyzer having a plurality of units necessary for analyzing a component contained in a sample; and a mobile terminal having an image capturing unit that captures, when a user holds the mobile terminal over one of the plurality of units, an image of the one unit and a display unit that displays, when the image capturing unit captures an image, a plurality of options for the user to perform next with respect to the one unit.

## Description

### Technical Field

The present invention relates to an automatic analysis system and a mobile terminal.

### Background Art

An automatic analyzer performs qualitative and quantitative analysis by the addition and reaction of a reagent that reacts specifically to a specific component contained in a sample such as blood and urine, and a measurement of the absorbance or the amount of luminescence of a reaction solution. In such an automatic analyzer, regular maintenance is performed to ensure the reliability of a measurement result. Thus, a known technique allows a user to display optimum information on a screen through a portable terminal, so that an unskilled user can easily perform operations such as maintenance. For example, Patent Literature 1 discloses that when a portable terminal is placed on identification means such as an RFID or a barcode on each functional unit of an automatic analyzer, the portable terminal retrieves identification information and combines the information with an operator ID, so that the portable terminal displays the maintenance date and time of the functional unit and necessary points of an operation manual.

### Citation List

### Patent Literature

Patent Literature 1: Japanese Unexamined Patent Application Publication No. 2013-64674

### Summary of Invention

### Technical Problem

In the technique described in Patent Literature 1, the placement of a mobile terminal on the identification means of a target unit is necessary as well as a modification to hardware for installing the identification means.

An object of the present invention is to provide an automatic analysis system and a mobile terminal that can display user-requested information with simpler operations without making significant changes to the hardware configuration of an analyzer.

### Solution to Problem

In order to attain the object, an automatic analysis system of the present invention includes: an automatic analyzer including a plurality of units necessary for analyzing a component contained in a sample; an image capturing unit that captures an image of one of the units when a user holds one of the units over the image capturing unit; and a display unit that displays a plurality of options to be subsequently performed by the user for the unit when the image capturing unit captures the image.

Moreover, a mobile terminal of the present invention includes: an image capturing unit that captures an image of one of a plurality of units necessary for analyzing a component contained in a sample, when a user holds one of the units over the image capturing unit; and a display unit that displays a plurality of options to be subsequently performed by the user for the unit when the image capturing unit captures the image. Advantageous Effects of Invention

The present invention can provide an automatic analysis system and a mobile terminal that can display user-requested information with simpler operations without making significant changes to the hardware configuration of an analyzer.

### Brief Description of Drawings

[Fig. 1] Fig. 1 illustrates an example of the configuration of an automatic analysis system according to a first embodiment.
[Fig. 2] Fig. 2 is a block diagram illustrating the functional configuration of an analyzer PC, an administrative server, and a mobile terminal in the automatic analysis system according to the first embodiment.
[Fig. 3] Fig. 3 illustrates an example of a data configuration stored in the storage unit of the analyzer PC.
[Fig. 4] Fig. 4 illustrates an example of operation information stored in the storage unit of the analyzer PC.
[Fig. 5] Fig. 5 illustrates an example of a data configuration stored in the storage unit of the administrative server.
[Fig. 6] Fig. 6 is a block diagram illustrating the detail of the functional configuration of the mobile terminal.
[Fig. 7] Fig. 7 is a flowchart showing an example of a processing flow before information is displayed on the mobile terminal.
[Fig. 8] Fig. 8 shows an example of a screen output by the display unit of the mobile terminal in a state in which a camera is held over a sample dispensing mechanism.
[Fig. 9] Fig. 9 shows an example of a screen output by the display unit of the mobile terminal when the sample dispensing mechanism is recognized.
[Fig. 10] Fig. 10 shows an example of a screen displayed when maintenance information is selected on the screen of Fig. 9.
[Fig. 11] Fig. 11 shows an example of a screen displayed when operation information is selected on the screen of Fig. 9.
[Fig. 12] Fig. 12 shows an example of a screen displayed when maintenance manual information is selected on the screen of Fig. 9.
[Fig. 13] Fig. 13 shows an example of a screen output by the display unit of the mobile terminal when a reagent disk is recognized.
[Fig. 14] Fig. 14 shows an example of a screen displayed when reagent erection information is selected on the screen of Fig. 13.
[Fig. 15] Fig. 15 illustrates an example of the configuration of an automatic analysis system according to a second embodiment.
[Fig. 16] Fig. 16 is a block diagram illustrating the functional configuration of an analyzer PC and a mobile terminal in an automatic analysis system according to a third embodiment.

### Description of Embodiments

Embodiments of the present invention will be described below in accordance with the accompanying drawings.

### First Embodiment

### <Configuration example of automatic analysis system>

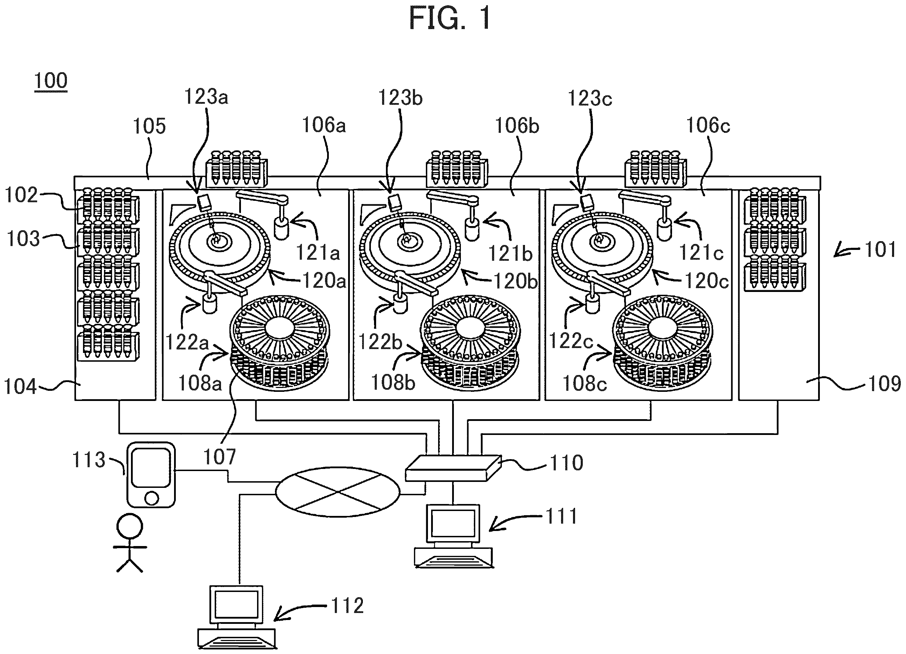

Fig. 1 illustrates an example of the configuration of an automatic analysis system according to a first embodiment. As illustrated in Fig. 1, an automatic analysis system 100 of the present embodiment includes an automatic analyzer, an administrative server 112, and a mobile terminal 113. The automatic analyzer includes an analysis operation unit 101, an analyzer PC 111, and an interface 110. The analyzer PC 111 is connected to the analysis operation unit 101 via the interface 110 and controls the analysis operation unit 101. The analyzer PC 111, the administrative server 112, and the mobile terminal 113 are connected to one another via a network.

The analysis operation unit 101 of the automatic analyzer includes a sample loading unit 104, analysis units 106a, 106b, and 106c, a conveyor line 105, and a sample storage unit 109. The sample loading unit 104 and the sample storage unit 109 are connected via the conveyor line 105 that conveys sample racks 103 (sample placement units). The sample loading unit 104 is loaded with the sample racks 103. The sample rack 103 is loaded with multiple sample containers 102, each containing a sample to be analyzed. The sample storage unit 109 stores the sample racks 103. In the present embodiment, the sample containers 102 are loaded in the sample racks 103. The sample containers 102 may be loaded in other sample placement units such as sample disks.

The analysis units 106a, 106b, and 106c are disposed along the conveyor line 105. In the example of Fig. 1, the three analysis units 106a, 106b, and 106c are disposed. The number of analysis units is not limited if one or more analysis units are provided.

The sample loading unit 104 is loaded with the sample racks 103, each being loaded with the multiple sample containers 102 containing samples to be analyzed. The sample racks 103 are conveyed to the analysis units 106a, 106b, and 106c and the sample storage unit 109 via the conveyor line 105.

Samples stored in the sample containers 102 include a general sample that is a biological specimen such as patient's blood or urine, a calibration sample used for calibration measurement, an accuracy control sample used for accuracy control measurement, a retest sample that is a sample for retesting, and an urgent sample to be analyzed prior to general samples.

The sample containers 102 placed in the sample rack 103 have tags that identify contained samples. The tags are not illustrated. For example, the tags are barcodes or RFIDs. Identification information is read by a reader, which is not illustrated, and is transmitted to the analyzer PC 111.

The analysis unit 106a includes a reaction disk 120a (incubator), a sample dispensing mechanism 121a, a reagent disk 108a (reagent placement unit), a reagent dispensing mechanism 122a, and a photometric mechanism 123a. The reaction disk 120a has a plurality of reaction containers.

The sample dispensing mechanism 121a sucks a sample contained in the sample container 102 conveyed by the conveyor line 105 and dispenses the sample into the reaction container. The reagent disk 108a is loaded with multiple reagent containers 107 (reagent bottles) that contain reagents used for sample analysis.

The reagent dispensing mechanism 122a sucks the reagent of the reagent container 107 loaded on the reagent disk 108a and dispenses the reagent into the reaction container. The photometric mechanism 123a measures the absorbance and scattered light intensity or the like of a mixed solution of the sample and the reagent that are contained in the reaction container.

The analysis unit 106a can analyze, for example, the concentration of a predetermined substance contained in the sample by analyzing optical information obtained from the photometric mechanism 123a. The analysis units 106b and 106c also have the same configuration as the analysis unit 106a and include reaction disks 120b and 120c, sample dispensing mechanisms 121b and 121c, reagent disks 108b and 108c, reagent dispensing mechanisms 122b and 122c, and photometric mechanisms 123b and 123c, respectively.

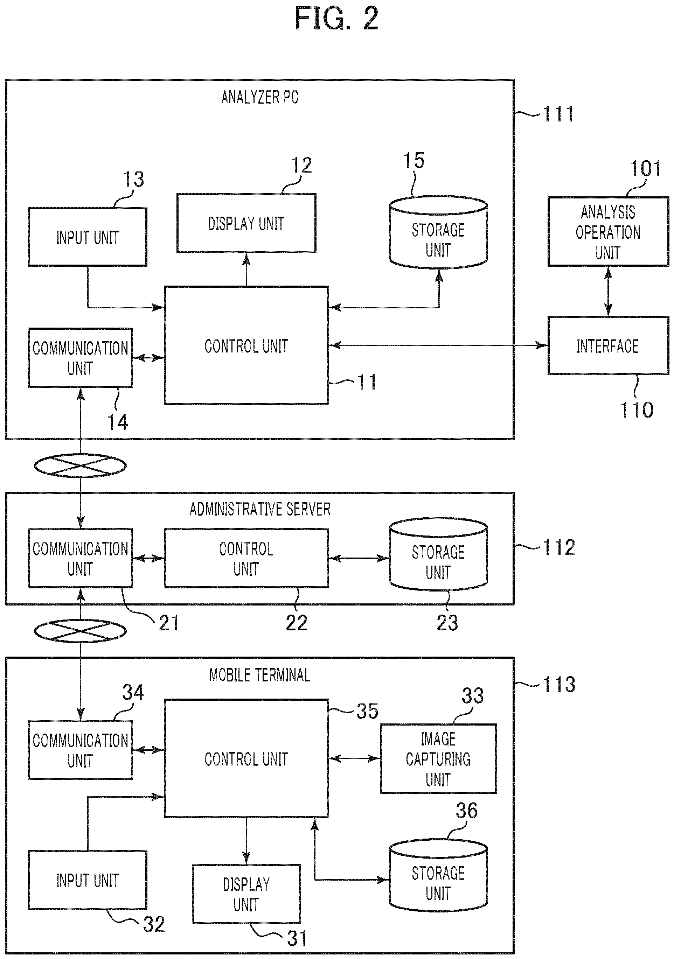

Fig. 2 is a block diagram illustrating the functional configuration of the analyzer PC, the administrative server, and the mobile terminal in the automatic analysis system according to the first embodiment.

### <Analyzer PC>

As illustrated in Fig. 2, the analyzer PC 111 includes a control unit 11, a display unit 12, an input unit 13, a communication unit 14, and a storage unit 15. The control unit 11 controls the operations of units constituting the analysis operation unit 101 via the interface 110 and conducts analysis corresponding to an item in response to a signal from the photometric mechanism. The display unit 12 is, for example, a display that outputs various kinds of information to a user. The input unit 13 is, for example, a keyboard or a mouse. The input unit 13 presses a button display with a mouse cursor on various operation screens displayed on the display unit 12. For example, a mouse is clicked or numbers or characters are input into an entry field from a keyboard. The communication unit 14 transmits various kinds of information stored in the storage unit 15 to the administrative server 112.

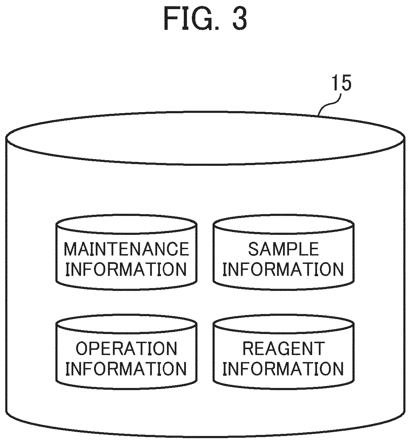

Fig. 3 illustrates an example of a data configuration stored in the storage unit of the analyzer PC. As illustrated in Fig. 3, sample information, reagent information, maintenance information, and operation information are stored in the storage unit 15. The sample information includes a sample ID, a patient name, and a measurement result.

The reagent information includes a reagent type, a remaining reagent amount, and a bottle loading date (replacement date). The maintenance information includes a previous maintenance date and a maintenance expiration date. The operation information includes the number of measurements for each analysis item and the total number of samples.

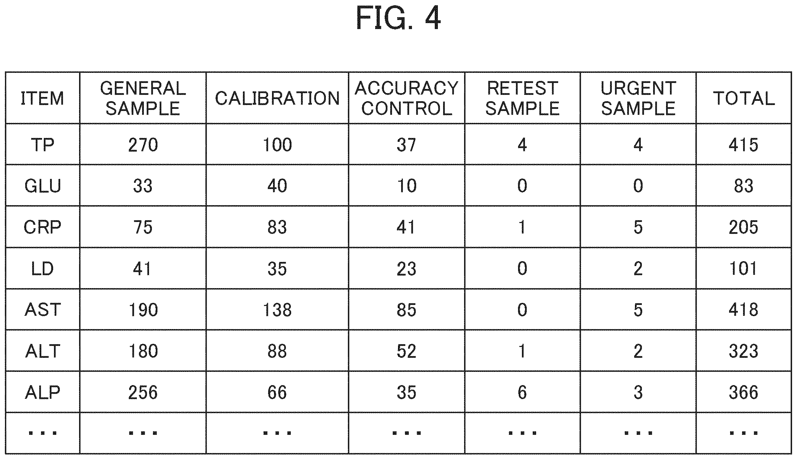

Fig. 4 illustrates an example of operation information stored in the storage unit of the analyzer PC. For measurement items such as TP, LD, and ALP, the number of measured samples is counted for each of a general sample, a calibration sample, an accuracy control sample, a retest sample, and an urgent sample and is stored as operation information in the storage unit 15. The operation information in Fig. 4 is obtained by counting the number of samples having undergone colorimetric analysis based on a luminous intensity measured by the photometric mechanism of each analysis unit. When the analysis unit measures an electrolyte, the number of samples (the number of measurements) for each analysis item may be counted for each sample type.

### <Administrative server>

As illustrated in Fig. 2, the administrative server 112 includes a communication unit 21, a control unit 22, and a storage unit 23. The communication unit 21 receives various kinds of information transmitted from the analyzer PC 111 and transmits various kinds of information stored in the storage unit 23 and information processed in the administrative server 112 to the mobile terminal 113. The control unit 22 stores, in the storage unit 23, various kinds of information received from the analyzer PC 111 and performs functions by reading programs stored in the storage unit 23. The administrative server 112 may be a server constructed with a single computer or a cloud server constructed with multiple computers.

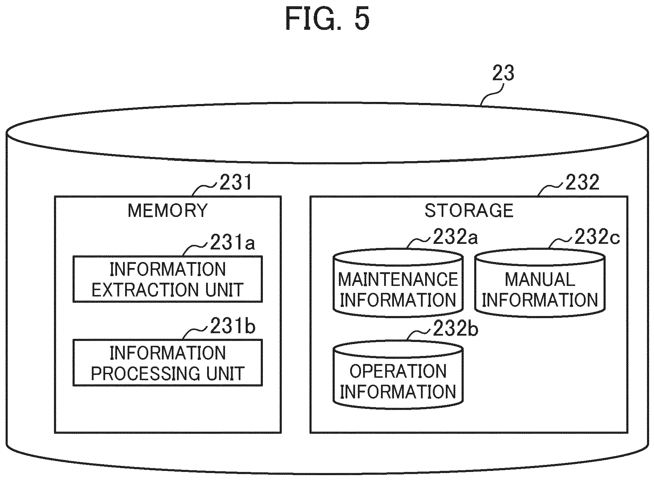

Fig. 5 illustrates an example of a data configuration stored in the storage unit of the administrative server. As illustrated in Fig. 5, the storage unit 23 of the administrative server 112 includes a memory 231 and a storage 232.

In the memory 231, programs corresponding to functions executed by the control unit 22 (processor) are stored as an information extraction unit 231a and an information processing unit 231b. The information extraction unit 231a extracts information from the storage 232 in response to a request from the mobile terminal 113. The information processing unit 231b uses the information extracted by the information extraction unit 231a and processes the information for transmission to the mobile terminal 113.

The programs to be provided or distributed may be preinstalled in ROM or the like or may be recorded as a file in an installable format or in an executable format in a recording medium, e.g., CD-ROM readable by a computer. Furthermore, the programs to be provided or distributed may be stored on a computer connected to a network, e.g., the Internet and may be downloaded via the network.

The storage 232 is composed of a nonvolatile storage device and includes a maintenance information database 232a, an operation information database 232b, and a manual information database 232c. In the maintenance information database 232a, pieces of information manually input by a user through the mobile terminal 113 as necessary are sequentially stored in association with the ID of the automatic analyzer in addition to operation information automatically transmitted from the analyzer PC 111. The information manually input by a user includes, for example, dates of manual maintenance such as cleaning of the sample dispensing mechanism, the reagent dispensing mechanism, and the reagent disk. Also in the operation information database 232b, pieces of operation information or the like automatically transmitted from the analyzer PC 111 are sequentially stored in association with the ID of the automatic analyzer. In the manual information database 232c, a maintenance manual is stored for each model of automatic analyzers. Maintenance manuals are sorted into the files of units and maintenance contents. Each of the files contains moving images indicating actual maintenance operations as well as a description about a maintenance procedure. In the storage 232, for example, a reagent erection information database that stores a remaining reagent amount and a bottle replacement date may be included as another database.

### <Mobile terminal>

The mobile terminal 113 is, for example, a smartphone or a tablet terminal owned by a user.

In this case, users in the present specification include an administrator or a serviceperson of the automatic analyzer as well as an operator of the automatic analyzer. The user can obtain information about the automatic analyzer by operating the mobile terminal 113 to make access to the administrative server 112.

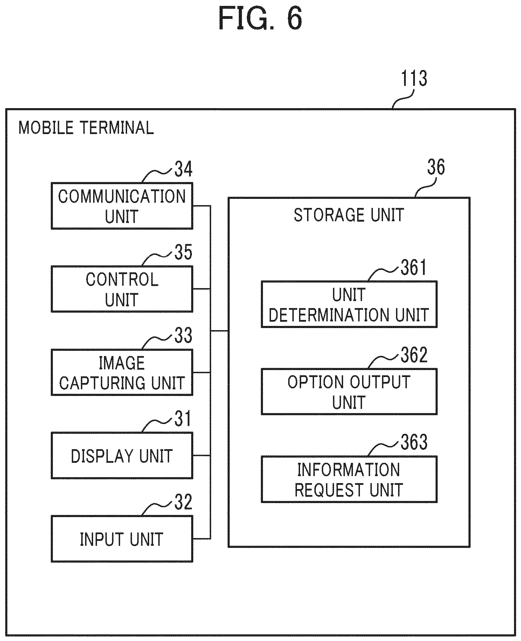

Fig. 6 is a block diagram illustrating the detail of the functional configuration of the mobile terminal. The mobile terminal 113 includes a display unit 31, an input unit 32, an image capturing unit 33, a communication unit 34, a control unit 35, and a storage unit 36.

The display unit 31 is a display that outputs various kinds of information to a user.

The input unit 32 is a touch sensor that is provided on the surface of a display and allows the user to input various kinds of information. The image capturing unit 33 is a camera that captures an image in response to an operation by the input unit 32. An image being captured and a captured image are outputted to the display unit 31. The communication unit 34 makes an inquiry to ask the administrative server 112 for transmission of predetermined information and receives the information transmitted from the administrative server 112 in response to the inquiry.

The control unit 35 performs functions by reading programs stored in the storage unit 36. In Fig. 6, the functions performed by the control unit 35 (processor) are conceptually illustrated as a unit determination unit 361, an option output unit 362, and an information request unit 363. The unit determination unit 361 determines which one of the units of the analysis operation unit 101 is held below the mobile terminal on the basis of an image captured by the image capturing unit 33. The option output unit 362 outputs, to the display unit 31, multiple options to be subsequently performed by the user for the unit determined by the unit determination unit 361. The options output by the option output unit 362 are determined in advance for the respective units. For example, when the sample dispensing mechanism is selected, maintenance control, operation information, and maintenance manual information are output as options. The information request unit 363 transmits an inquiry to ask for transmission of information about the specified option, to the administrative server 112 via the communication unit 34. The programs are stored on a computer (e.g., an administrative server) connected to a network and are provided by downloading via the network.

### <Processing flow before information is displayed on mobile terminal>

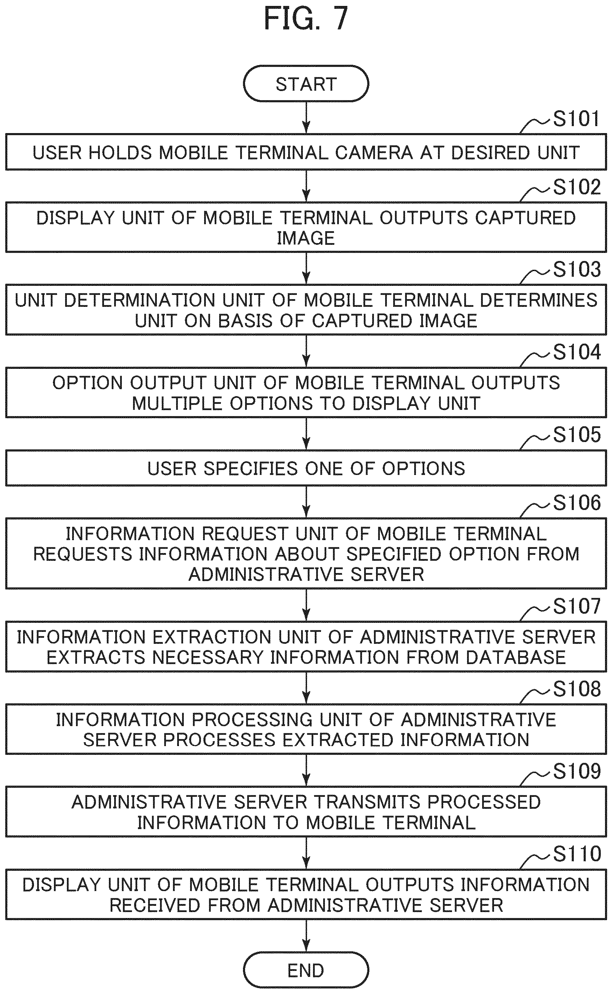

Fig. 7 is a flowchart showing an example of a processing flow before information is displayed on the mobile terminal. First, the user performs processing for specifying the automatic analyzer having desired information by using the mobile terminal 113. For example, through the mobile terminal 113, the user makes access to a preinstalled predetermined application or a web site provided by the administrative server or the like and inputs the user ID through the input unit 32. At this point, a preregistered candidate of the automatic analyzer is output to the display unit 31 in association with the user ID. When the user selects the predetermined automatic analyzer through the input unit 32, the automatic analyzer is set as a target of confirmation.

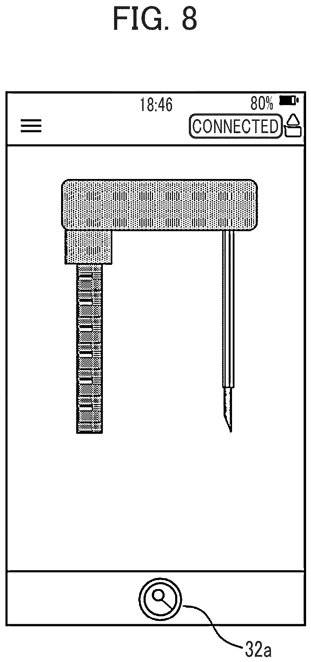

The image capturing unit 33 is then automatically or manually activated, and the user holds the camera over desired one of the units constituting the analysis operation unit 101 of the automatic analyzer (step S101). Fig. 8 shows an example of a screen output by the display unit of the mobile terminal in a state in which the camera is held over the sample dispensing mechanism that is one of the units. As shown in Fig. 8, when the user taps a predetermined determination button 32a in a state in which one of the units is included in the imaging range of the camera, the display unit 31 outputs a captured image upon determination, and the image capturing unit 33 stores the captured image in the storage unit 36 (step S102). The unit determination unit 361 then determines the unit on the basis of the captured image (step S103). When the unit determination unit 361 outputs a predetermined unit on the basis of the input image, a prepared learning model may be used or the learning model may be prepared for each type of the automatic analyzer.

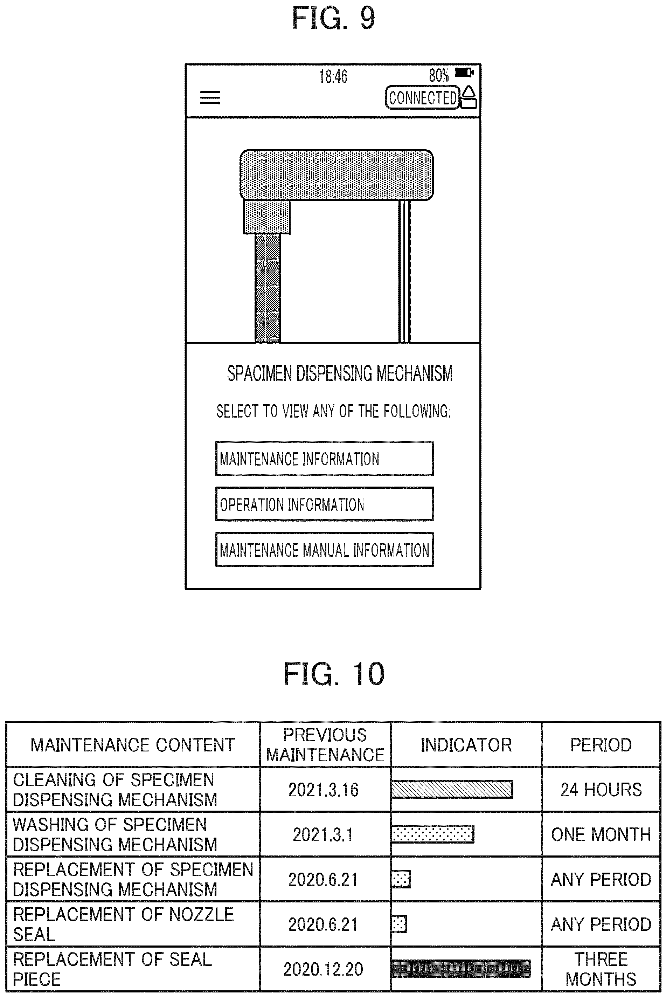

Thereafter, the display unit 31 outputs character information (e.g., a unit name) about the unit determined by the unit determination unit 361, with the image captured upon determination. The output of the captured image allows the user to confirm the determined unit, and the output of the character information provides a notification to the user when a wrong imaging target is selected. Furthermore, the option output unit 362 causes the display unit 31 to output predetermined multiple options for the unit and provides display that prompts the user to make a choice (step S104). Fig. 9 shows an example of a screen output by the display unit of the mobile terminal when the sample dispensing mechanism is recognized. For example, when the sample dispensing mechanism is recognized, as shown in Fig. 9, the option output unit 362 outputs three options: maintenance information that is information about the maintenance of the sample dispensing mechanism, operation information that is information about the operation condition of the sample dispensing mechanism, and maintenance manual information about the sample dispensing mechanism. The options output by the option output unit 362 are not limited to the three options. Any two of the options may be output or other options may be included.

When the user specifies one of the options through the input unit 32 (step S105), the information request unit 363 transmits an inquiry to ask for transmission of information about the specified option with the ID of the automatic analyzer to be confirmed, to the administrative server 112 via the communication unit 34 (step S106). When the communication unit 21 of the administrative server 112 receives the inquiry from the mobile terminal 113 and the ID of the automatic analyzer, the information extraction unit 231a extracts necessary information for the option specified in the inquiry from databases in the storage 232 (step S107). At this point, the information processing unit 231b uses primary information extracted by the information extraction unit 231a and performs additional calculation or graphing to process the primary information into secondary information for transmission to the mobile terminal 113 (step S108).

Thereafter, the communication unit 21 of the administrative server 112 transmits the secondary information to the mobile terminal 113 corresponding to the user ID (step S109). The mobile terminal 113 outputs the secondary information received from the administrative server 112 to the display unit 31 (step S110).

Fig. 10 shows an example of a screen displayed when the maintenance information is selected on the screen of Fig. 9. The following describes a method in which the administrative server 112 processes primary information in the storage unit 23 into secondary information included in the screen of Fig. 10. First, the information extraction unit 231a of the administrative server 112 extracts, from the maintenance information database 232a, a previous maintenance date and a maintenance expiration date (maintenance period) for each maintenance content of the sample dispensing mechanism. The maintenance expiration date may be determined in advance for each automatic analyzer or may be set by the user as appropriate. Subsequently, the information processing unit 231b of the administrative server 112 visually outputs the current state in an indicator form on the basis of the previous maintenance date and the maintenance expiration date. Colors may be selectively output according to the state. For example, red may be output after the maintenance expiration date, or yellow may be output just before the subsequent maintenance date.

Fig. 10 is merely an exemplary screen of maintenance information when the sample dispensing mechanism is recognized. Also when other units are recognized, a cleaning or washing date, the presence or absence/necessity or unnecessity of a maintenance operation, or the necessity or unnecessity of replacement is output to the display unit upon the selection of the maintenance information. Also on the screen of maintenance information about other units, display is provided to notify a user about a maintenance item if a recommended maintenance date for the maintenance item has passed.

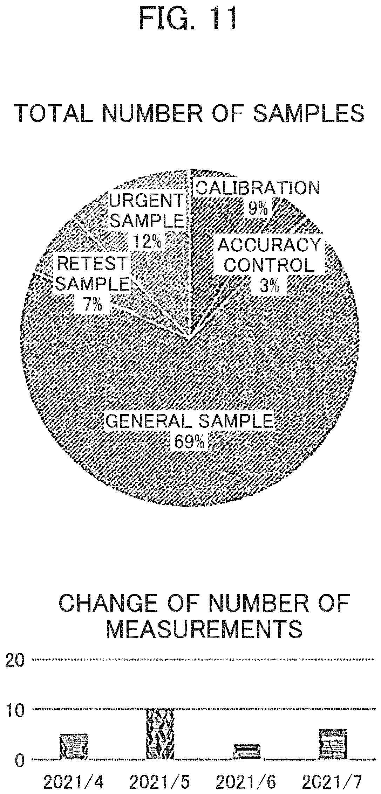

Fig. 11 shows an example of a screen displayed when the operation information is selected on the screen of Fig. 9. The following describes a method in which the administrative server 112 processes primary information in the storage unit 23 into secondary information included in the screen of Fig. 11. In the screen of Fig. 11, the total number of samples is shown as a graph of primary information extracted from the operation information database 232b by the information extraction unit 231a, the graph being made by the information processing unit 231b. On the other hand, regarding a change of the number of measurements, first, the information extraction unit 231a extracts primary information stored in the operation information database 232b, the primary information including the number of measurements for each analysis item. The information processing unit 231b then calculates the number of measurements in each predetermined period on the basis of the extracted primary information and plots a graph of the calculation result.

Fig. 11 is merely an exemplary screen of operation information when the sample dispensing mechanism is recognized. Also when other units are recognized, the number of measurements for each analysis item, the total number of samples, a remaining amount of the reagent, or a reagent expiration date is output to the display unit 31 upon the selection of the operation information. When an incubator is recognized, the use history of a reaction container to be placed on the incubator is output to the display unit upon the selection of the operation information. When a calibration sample rack is recognized, the calibration result may be output, whereas when an accuracy control sample rack is recognized, the accuracy control result may be output.

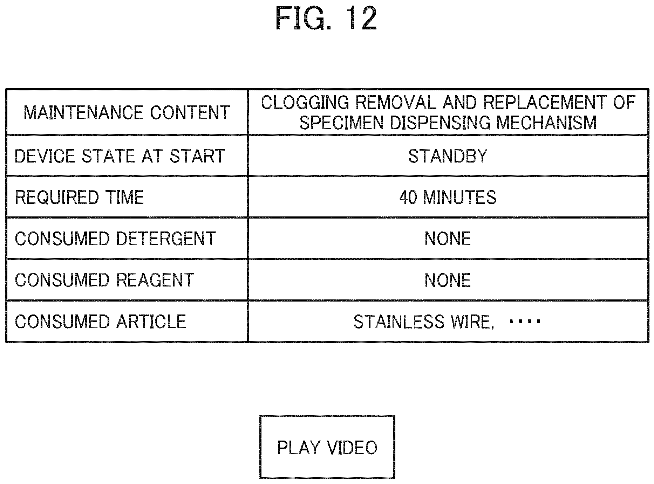

Fig. 12 shows an example of a screen displayed when the maintenance manual information is selected on the screen of Fig. 9. From among pieces of information included in the screen of Fig. 12, character information about the outline of maintenance is extracted from the manual information database 232c by the information extraction unit 231a of the administrative server 112 and is transmitted via the communication unit 21. Furthermore, among pieces of information included in the screen of Fig. 12, only the URL of the manual information database 232c in which video data is stored for video replay. Video can be replayed by user access to the URL. The character information about the outline of maintenance may also be viewable by user access to a predetermined URL.

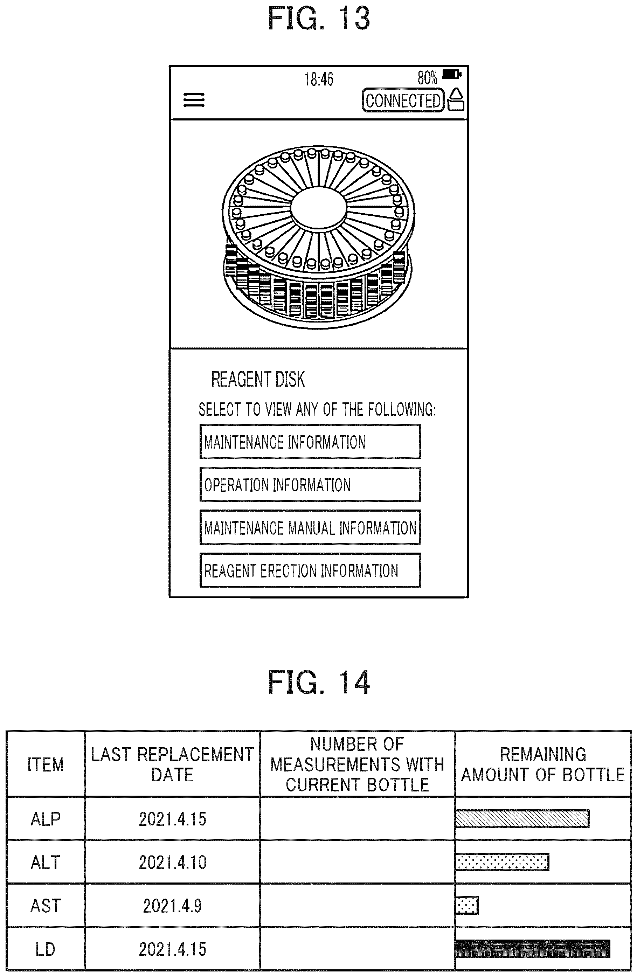

Fig. 13 shows an example of a screen output by the display unit of the mobile terminal when the reagent disk is recognized. For example, when the reagent disk is recognized, the option output unit 362 outputs four options: maintenance information that is information about the maintenance of the reagent disk, operation information that is information about the operation condition of the reagent disk, maintenance manual information about the reagent disk, and reagent erection information.

Fig. 14 shows an example of a screen displayed when the reagent erection information is selected on the screen of Fig. 13. The screen of the reagent erection information includes the indicator of a bottle remaining amount and information about the number of measurements of the current bottle in addition to a final replacement date for each reagent bottle.

The indicator of a bottle remaining amount is obtained by processing primary information about a bottle remaining amount into secondary information in an indicator form by the information processing unit 231b, the primary information being sequentially transmitted from the analyzer PC 111 and stored in the database of the administrative server 112. The number of measurements of the current bottle is obtained by subtracting the number of measurements of the reagent on the final replacement date of the reagent bottle from the number of measurements of the reagent at the present time by the information processing unit 231b. In other words, the administrative server 112 processes primary information, which is not directly stored in the storage unit 15 of the analyzer PC 111, into secondary information useful for the user, and provides the secondary information to the mobile terminal 113.

In Fig. 13, the option of reagent erection information is provided in addition to the option of operation information. The option may be included in the option of operation information. In other words, a screen displayed as reagent erection information in Fig. 14 may be included in a screen displayed when the option of operation information is selected.

According to the present embodiment, the user of the automatic analyzer including multiple units can display an image of the unit having desired information on the display unit 31 on the terminal only by holding the mobile terminal 113 over the unit without the need for placing the mobile terminal 113 onto the unit. Thus, the user can quickly recognize that the desired unit has been selected from the multiple units. Moreover, the unit is recognized by capturing an image, thereby eliminating the need for hardware modification such as embedding of an RFID or a barcode. Furthermore, information only about the unit of a captured image is retrieved. This can eliminate the need for storing information about unnecessary units in the mobile terminal 113 and reduce the traffic of the mobile terminal 113. The user is prompted to select necessary information to be obtained, and the information is obtained by a simple operation without manual input of text, thereby saving user's work as compared with search for information on a normal PC screen.

### Second Embodiment

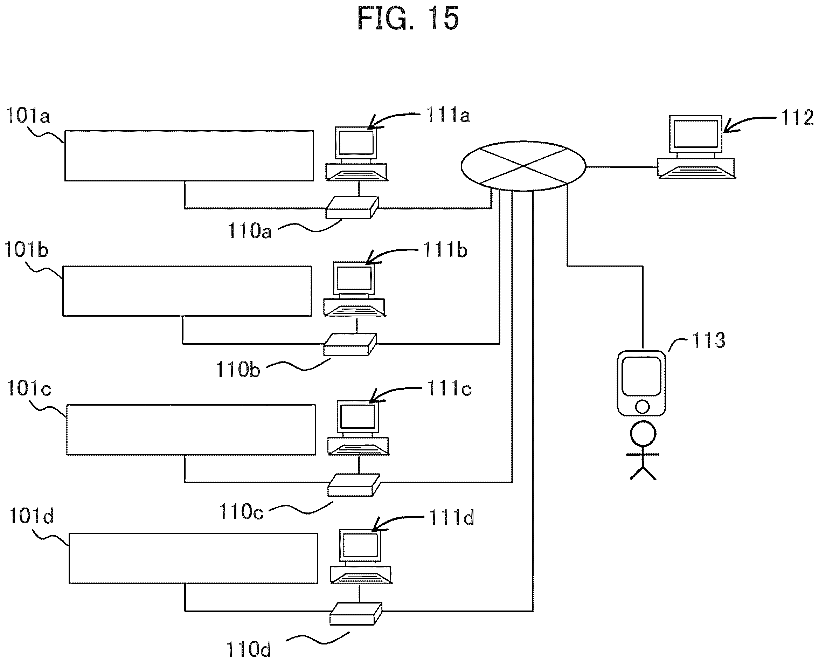

Fig. 15 illustrates an example of the configuration of an automatic analysis system according to a second embodiment. Like the first embodiment, the automatic analysis system of the present embodiment includes multiple automatic analyzers in the same facility.

In the example of Fig. 15, analyzer PCs 111a to 111d of four automatic analyzers are connected to an administrative server 112. The number of connected analyzers is not limited thereto.

In the present embodiment, when a user holds a mobile terminal 113 over a desired unit of any one of the automatic analyzers, a display unit 31 provides display to prompt the user to select the target automatic analyzer. When the user selects the automatic analyzer, the preregistered candidates of automatic analyzers may be displayed on a display unit 31 in association with the user ID, or an image capturing unit 33 may capture an image of a serial number or a device ID to specify the automatic analyzer through image recognition.

### Third Embodiment

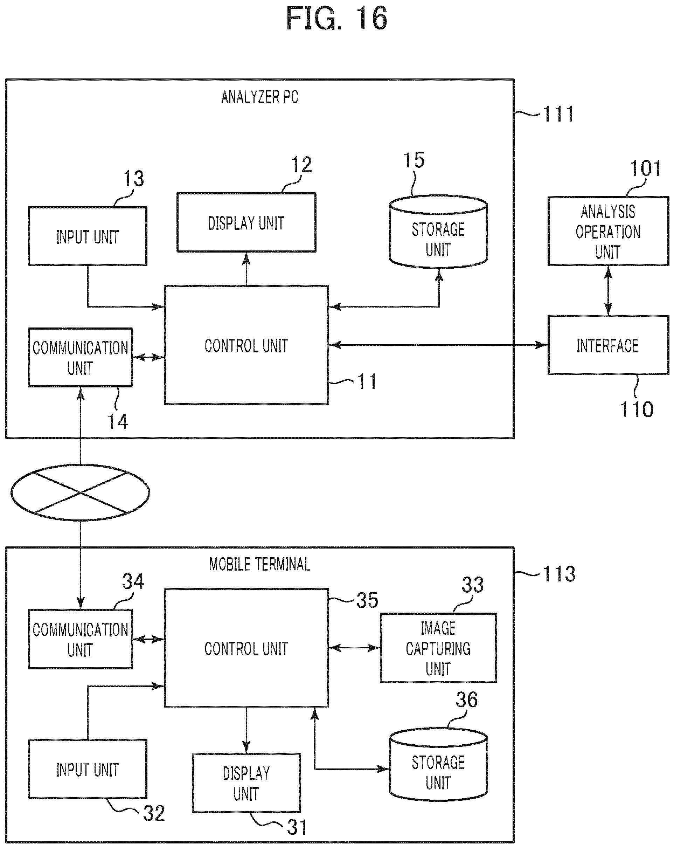

Fig. 16 is a block diagram illustrating the functional configuration of an analyzer PC and a mobile terminal in an automatic analysis system according to a third embodiment. Unlike in the first and second embodiments, an automatic analysis system of the present embodiment allows a mobile terminal 113 to directly exchange data with an analyzer PC 111 without an administrative server.

In the present embodiment, communication connection between the mobile terminal 113 and the analyzer PC 111 requires authentication (pairing) processing between the mobile terminal 113 and the analyzer PC 111. At the time of paring, a user connects the mobile terminal 113 to the analyzer PC 111 through a network via a wireless LAN or directly connects the mobile terminal 113 to the analyzer PC 111 through near-field radio communications using Bluetooth (registered trademark) or the like.

Also in the present embodiment, when the mobile terminal 113 transmits, to the analyzer PC 111, an inquiry to ask for transmission of information about a specified option, requested information can be received from the analyzer PC 111. Furthermore, in the present embodiment, the analyzer PC 111 does not need to sequentially transmit data including operation information to an administrative server 112, thereby suppressing the traffic of data.

Various modifications may be adopted in addition to the foregoing embodiments. For example, when the user holds the mobile terminal 113 over a unit, a display unit 31 provides display to prompt the user to input specific information (ID or password). When the user inputs the specific information to perform a login, user-specific information for the user may be displayed. The user-specific information includes at least first information about an operator serving as the user, second information about an administrator serving as the user, and third information about a serviceperson serving as the user. It is desirable to minimize the amount of the first information and maximize the amount of third information.

The method for displaying multiple options is not limited to the methods of Figs. 9 and 13. In the case of a large number of options, the options may also be output in a pull-down menu. Moreover, if the mobile terminal 113 does not include the option output unit, the administrative server 112 may transmit options to the mobile terminal 113. Moreover, if the mobile terminal 113 does not include the unit determination unit, image data acquired by the image capturing unit 33 may be transmitted to the administrative server 112, and then the administrative server 112 may determine the unit. The information processing unit 231b of the administrative server 112 may perform information processing at the time of transmission of information from the analyzer PC 111 to the administrative server 112 instead of the transmission of an inquiry to ask for transmission of information from the mobile terminal 113.

The foregoing embodiments are described in detail for the sake of clarity of the present invention. The present invention is not limited to an embodiment including all the foregoing configurations. Furthermore, the configuration of one embodiment may be partially replaced with the configuration of another embodiment, or the configuration of one embodiment may be added to the configuration of another embodiment. Moreover, the configurations of the embodiments may additionally include other configurations, may be partially deleted, or may be partially replaced with other configurations.

### List of Reference Signs

11: control unit (analyzer PC)
12: display unit (analyzer PC)
13: input unit (analyzer PC)
14: communication unit (analyzer PC)
15: storage unit (analyzer PC)
21: communication unit (administrative server)
22: control unit (administrative server)
23: storage unit (administrative server)
31: display unit (mobile terminal)
32: input unit (mobile terminal)
32a: determination button
33: image capturing unit
34: communication unit (mobile terminal)
35: control unit (mobile terminal)
36: storage unit (mobile terminal)
100: automatic analysis system
101, 101a to 101d: analysis operation unit
102: sample container
103: sample rack
104: sample loading unit
105: conveyor line
106a to 106c: analysis unit
107: reagent container
108a to 108c: reagent disk
109: sample storage unit
110, 110a to 110d: interface
111, 111a to 111d: analyzer PC
112: administrative server
113: mobile terminal
120a to 120c: reaction disk
121a to 121c: sample dispensing mechanism
122a to 122c: reagent dispensing mechanism
123a to 123c: photometric mechanism
231: memory
231a: information extraction unit
231b: information processing unit
232: storage
232a: maintenance information database
232b: operation information database
232c: manual information database
361: unit determination unit
362: option output unit
363: information request unit

## Claims

1. An automatic analysis system comprising:
an automatic analyzer including a plurality of units necessary for analyzing a component contained in a sample;
an image capturing unit that captures an image of one of the units when a user holds one of the units over the image capturing unit; and
a mobile terminal including a display unit that displays a plurality of options to be subsequently performed by the user for the unit when the image capturing unit captures the image.

2. The automatic analysis system according to claim 1,
wherein when the user selects the first option of the plurality of options, the display unit provides display to prompt the user to select at least one of maintenance information about maintenance of the unit and operation information about an operation condition of the unit.

3. The automatic analysis system according to claim 1, further comprising a server that performs information communications with the mobile terminal,
wherein when the user holds the mobile terminal over the first unit of the plurality of units, the mobile terminal transmits, to the server, an inquiry to ask for transmission of information about the first unit, and
the server transmits the information about the first unit to the mobile terminal in response to the inquiry.

4. The automatic analysis system according to claim 2,
wherein when the user selects the maintenance information, the display unit displays at least a cleaning or washing date and time of the unit, presence or absence/necessity or unnecessity of a maintenance operation, or necessity or unnecessity of replacement.

5. The automatic analysis system according to claim 2,
wherein the display unit provides display to notify a user about a maintenance item if a recommended maintenance date for the maintenance item has passed when the user selects the maintenance information.

6. The automatic analysis system according to claim 4,
wherein the unit is at least one of a sample dispensing mechanism that sucks/dispenses the sample, a sample placement unit for loading the sample, a reagent dispensing mechanism that sucks/dispenses a reagent, and a reagent placement unit for placing the reagent, and
the operation information includes at least the number of measurements for each analysis item, a total number of the samples, or a remaining amount of the reagent.

7. The automatic analysis system according to claim 2,
wherein the unit is an incubator, and
the operation information includes at least use history of a reaction container to be placed on the incubator.

8. The automatic analysis system according to claim 1,
wherein when the user holds the mobile terminal over the unit, the display unit provides display to prompt the user to input specific information, and when the user inputs the specific information, the display unit displays user-specific information for the user.

9. The automatic analysis system according to claim 8,
wherein the user-specific information includes at least information about an operator serving as the user and information about a serviceperson serving as the user.

10. The automatic analysis system according to claim 1, further comprising a first automatic analyzer and a second automatic analyzer,
wherein when the user holds the mobile terminal over the unit, the display unit provides display to prompt the user to select one of the first automatic analyzer and the second automatic analyzer.

11. The automatic analysis system according to claim 1,
wherein when the image capturing unit captures an image, the display unit displays the captured image.

12. The automatic analysis system according to claim 11,
wherein the display unit displays character information about the unit with the captured image.

13. A mobile terminal comprising:
an image capturing unit that captures an image of one of a plurality of units necessary for analyzing a component contained in a sample, when a user holds one of the units over the image capturing unit; and
a display unit that displays a plurality of options to be subsequently performed by the user for the unit when the image capturing unit captures the image.
